(19) Europäisches Patentamt

European Patent Office

Office européen des brevets

(11) Veröffentlichungsnummer: **0 307 747 B1**

(12) **EUROPÄISCHE PATENTSCHRIFT**

(45) Veröffentlichungstag der Patentschrift: **13.05.92**

(21) Anmeldenummer: **88114454.7**

(22) Anmeldetag: **05.09.88**

(51) Int. Cl.⁵: **C09C 1/00**

(54) **Goldfarbene Farbglanzpigmente.**

(30) Priorität: **11.09.87 DE 3730505**

(43) Veröffentlichungstag der Anmeldung:
**22.03.89 Patentblatt 89/12**

(45) Bekanntmachung des Hinweises auf die
Patenterteilung:
**13.05.92 Patentblatt 92/20**

(84) Benannte Vertragsstaaten:
**BE CH DE ES FR GB IT LI NL**

(56) Entgegenhaltungen:
**EP-A- 0 211 351**
**EP-A- 0 246 523**

(73) Patentinhaber: **MERCK PATENT GESELL-
SCHAFT MIT BESCHRÄNKTER HAFTUNG
Frankfurter Strasse 250 Postfach 4119
W-6100 Darmstadt(DE)**

(72) Erfinder: **Emmert, Ralf, Dr.
419 John F. Kennedy Bvd.
Bayonne, N.J. 07002(US)**
Erfinder: **Weigand, Manfred, Dr.
Im Hilsbruch 90
W-6100 Darmstadt(DE)**

**Beschreibung**

Die Erfindung betrifft Farbglanzpigmente auf Basis von mit einer Metalloxidschicht überzogenen plättchenförmigen Substraten, insbesondere Glimmer oder mit Metalloxiden überzogener Glimmer, wobei die Metalloxidschicht sowohl Titan als auch Eisen enthält.

Eisenhaltige Glimmerschuppenpigmente sind bekannt und werden auch seit vielen Jahren mit Erfolg angewendet. Dabei werden sowohl Pigmente beschrieben, bei denen Eisenoxid zusammen mit einem anderen Metalloxid, insbesondere Titandioxid, auf die Glimmerplättchen aufgefällt wird, als auch Pigmente, bei denen die Fällungen nacheinander erfolgen.

Im US-Patent 3 087 828 wird beschrieben, daß durch Abscheiden einer $Fe_2O_3$-Schicht auf eine $TiO_2$-Schicht goldfarbene Pigmente erhalten werden, die beim Glühen einen rötlichen Farbton annehmen. In der DE-PS 19 59 998 sind Pigmente beschrieben, die auf Glimmer zunächst eine Mischschicht von Titan- und Eisenoxid und darauf eine Deckschicht aus Titan- und/oder Zirkondioxid besitzen.

In der DE-PS 22 44 298 wird ein Verfahren zur Herstellung von goldfarbenen Perlglanzpigmenten beschrieben, bei dem ein mit $TiO_2$- und/oder $ZrO_2$-beschichtetes Glimmerpigment zunächst mit Eisen-(II)-hydroxid beschichtet wird, das dann zu $Fe_2O_3$ oxidiert wird.

Schließlich werden in der DE-OS 27 23 871 Glimmerpigmente beschrieben, die auf einer sehr dünnen $TiO_2$- oder $Al_2O_3$-Schicht eine dicke $Fe_2O_3$-Schicht tragen.

In der DE-PS 25 22 572 wurde auch schon vorgeschlagen, mit $TiO_2$ in der Rutilform beschichtete Glimmerpigmente mit einer zusätzlichen Deckschicht aus färbenden Metalloxiden zu versehen, wobei auch $Fe_2O_3$ genannt ist. Tatsächlich wurden auch solche Beschichtungen mit relativ geringen Eisenoxidmengen vorgenommen.

In der DE-OS 23 13 331 sind vorteilhafte eisenhaltige Pigmente beschrieben, bei denen das Eisenoxid in bestimmten definierten Kristallmodifikationen vorliegt.

Nach dem Verfahren der DE-OS 35 28 256 werden mit Titandioxid in der Rutilform belegte Glimmerpigmente mit einer relativ hohen Eisenoxidmenge beschichtet, so daß ein Dreischichtenaufbau aus Rutil, Pseudobrookit und Eisenoxid entsteht, der sich durch die Röntgenstrukturanalyse nachweisen läßt.

Die bisher bekannten goldfarbenen Farbglanzpigmente auf der Basis von mit Metalloxid beschichtetem Glimmer weisen eine mangelhafte chemische und thermische Beständigkeit in Emails und Glasuren auf oder sind wegen Zugabe toxischer Schwermetalle ungeeignet zur Verwendung in Kosmetika.

Es bestand daher die Aufgabe, neue Farbglanzpigmente sowie ein neues Verfahren zu ihrer Herstellung zu finden. Die Pigmente sollten vor allem hohe chemische und thermische Stabilität besitzen, um sie in silikatischen Schmelzflüssen verwenden zu können. Das Fehlen jeglicher toxischer Bestandteile sollte darüberhinaus auch ihre Anwendung in kosmetischen Artikeln erlauben.

Es wurde nun überraschenderweise gefunden, daß eine unter geeigneten Bedingungen auf plättchenförmigen Substraten aufgefällte Mischoxidschicht, die vorwiegend aus Pseudobrookit besteht, zu sehr stabilen Farbglanzpigmenten führt. Ihre hohe thermische und chemische Stabilität erlaubt es, sie in Glasuren und Emails einzusetzen.

Gegenstand der Erfindung sind daher Farbglanzpigmente auf Basis von mit einer Metalloxidschicht überzogenen plättchenförmigen Substraten, insbesondere Glimmer oder mit Metalloxiden überzogener Glimmer, wobei die Metalloxidschicht sowohl Titan als auch Eisen enthält, dadurch gekennzeichnet, daß die Metalloxidschicht im wesentlichen einen Pseudobrookit der Zusammensetzung $(Fe_2O)_x \cdot (TiO_2)$, worin x alle Werte zwischen 0,75 und 1,5 annehmen kann, enthält, und wobei in der Metalloxidschicht ggf. noch 0 bis 5 Gew.% Dotierstoffe und auf der Metalloxidschicht ggf. noch 0 bis 5 Gew.% einer Nachbeschichtung enthalten sind.

Gegenstand der Erfindung ist auch ein Verfahren zur Herstellung von Farbglanzpigmenten durch Beschichten von plättchenförmigen Substraten wie Glas, Glimmer oder mit Metalloxiden überzogener Glimmer mit einer Eisen und Titan enthaltenden Metalloxidschicht und anschließendes Glühen, das dadurch gekennzeichnet ist, daß man zu einer wäßrigen Suspension eines plättchenförmigen Substrats bei einer Temperatur zwischen 50 und 100 °C, einem pH-Wert zwischen 2,0 und 6,0 und gleichzeitig eine wäßrige Eisen(III)-salzlösung, eine wäßrige Titan(IV)-salzlösung und eine Base so zuführt, daß stets ein Molverhältnis von Eisen zu Titan wie 1,5-2,5 : 1 eingehalten wird und der pH-Wert während der gesamten Beschichtung nahezu konstant bleibt.

Weiterhin ist die Verwendung dieser Faglanzpigmente in Kunststoffen, Lacken, Kosmetika und silikatischen Schmelzflüssen, wie Emails und Glasuren, Gegenstand der Erfindung.

Überraschenderweise besitzen Pigmente fit solch einer homogenen Schicht aus Pseudobrookit sowohl deutlich verbesserte koloristische Eigenschaften (verbesserte Farbstärke und Brillanz und verbessertes Deckvermögen) als auch eine verbesserte chemische Beständigkeit, insbesondere in bezug auf die Beständigkeit in Schmelzflüssen für Glasuren und Email, sowie eine verbesserte Fotoaktivität. Dies äußert sich durch hohen Glanz und große Farbtreue nach dem Brennen. Während die bisher bekannten goldenen Farbglanzpigmente beim Brennen in Emails oder Glasuren verblaßten, ihre Farbe stumpf wurde und sich nach braungelb veränderte, werden mit den neuen goldfarbenen Farbglanzpigmenten glänzendere und echtem Gold farblich ähnlichere Emails und Glasuren erhalten.

Die guten Eigenschaften dieser Pseudobrookit-Schicht zeigen sich über den gesamten Bereich der Zusammensetzung $(Fe_2O_3)_x \cdot (TiO_2)$, worin x alle Werte zwischen 0,75 und 1,5 annehmen kann. Besonders bevorzugt ist jedoch die Zusammensetzung mit x = 1, die der stöchiometrisch exakten Pseudobrookit-Zusammensetzung $Fe_2TiO_5$ entspricht.

Es ist wesentlich, daß die neuen Farbglanzpigmente eine sehr glatte Oberfläche besitzen, die der glatten zu beschichtenden Oberfläche der eingesetzten Substrate entspricht. Aus diesem Grund lassen sich diese Farbglanzpigmente nur unter Einhaltung definierter Verfahrensbedingungen herstellen. Die Hydrolyse sollte deshalb bei möglichst konstanter Temperatur, konstantem pH-Wert und konstanter Zuflußgeschwindigkeit der Metallsalzlösung durchgeführt werden. Dabei ist es wesentlich, daß ein Überschuß von Metallionen in der Suspension vermieden wird. Es darf also pro Zeiteinheit nur eine solche Metallsalzmenge zur Hydrolyse zugeführt werden, die als hydratisiertes Metalloxid von der Substratoberfläche pro Zeiteinheit aufgenommen werden kann. Nur wenn verhindert wird, daß freie hydratisierte Metallionen oder Metalloxidteilchen, die nicht auf der Substratoberfläche gebunden sind, in der Suspension vorhanden sind, erhält man homogene Schichten von einheitlicher Schichtdicke.

Als Substrate zur Auffällung der Metalloxidschicht sind im Prinzip alle plättchenförmigen Materialien geeignet, die mit Metalloxiden beschichtet werden können. Beispielhaft können genannt werden Glas, Glimmer, wie z.B. Muskovit, Biotit, Phlogopit und Vermiculit, synthetische Glimmer und mit Metalloxiden überzogener Glimmer.

Vorzugsweise wird Muskovit eingesetzt, wobei helle, an färbenden Metallverbindungen arme Glimmersorten bevorzugt werden. In Spezialfällen sind zur Erzielung eines bestimmten Farbcharakters jedoch auch gefärbte Glimmersorten geeignet.

Besonders vorteilhafte Pigmente erhält man auch bei Verwendung von vorher mit vorzugsweise 1-5 Gew.-% $SiO_2$, $SnO_2$, $Al_2O_3$, $TiO_2$ und/oder $Fe_2O_3$ belegtem Glimmer.

Die vorzugsweise verwendeten Glimmerschuppen haben je nach der gewünschten späteren Anwendung des Farbglanzpigmentes einen Durchmesser von etwa 2-1000 $\mu$m, vorzugsweise 5-50 $\mu$m, und eine Dicke im Bereich von etwa 0,05-1, vorzugsweise etwa 0,1 $\mu$m. Glimmer, dessen Dicke 1 $\mu$m übersteigt, ist für die Farbglanzpigmente nach der vorliegenden Erfindung nicht geeignet. Die Glimmerschuppen sollen im Hinblick auf Dicke und Durchmesser möglichst einheitlich sein. Es ist deshalb zweckmäßig, den Glimmer nach dem Spalten und Vermahlen zu klassieren.

Das zu beschichtende Substrat wird in wäßriger Suspension vorgelegt, zweckmäßig in einer Konzentration von etwa 5-40 Gew.-%. Die Metallsalzlösungen werden gleichmäßig in die Suspension an einer oder mehreren Stellen eingeführt. Gegebenenfalls können die Metallsalzlösungen auch vor der Zugabe zur Substratsuspension im gewünschten Verhältnis vorgemischt werden. Die Eisensalzlösung wird vorzugsweise in einer Konzentration von etwa 0,1-4 Mol/l und die Titansalzlösung in einer Konzentration von etwa 0,05-2 Mol/l eingesetzt. Das Molverhältnis von Eisen zu Titan in den Metallsalzlösungen liegt bei 1,5 : 1 bis 2,5 : 1, bevorzugt bei 1,8 : 1 und insbesondere bei 2,0 : 1. Dabei erhält man Metalloxidschichten, die einen Eisengehalt, berechnet als $Fe_2O_3$, von 60-72 Gew.-%, bevorzugt 64-69 Gew.-%, und insbesondere von 67 Gew.-%, aufweisen.

Als Metallsalze können grundsätzlich alle wasserlöslichen Salze der genannten Metalle eingesetzt werden. Vorzugsweise werden die Chloride verwendet. Diese besitzen gegenüber den sonst auch noch leicht zugänglichen Sulfaten den Vorteil, daß das Chloridion im Metalloxidhydrat nicht so fest wie das Sulfation gebunden wird. Es kann daher leichter wieder ausgewaschen werden.

Die Metalloxide werden auf der Substratoberfläche niedergeschlagen, ohne daß Nebenfällungen auftreten. Vorzugsweise erreicht man dies, indem man die Zulaufgeschwindigkeit so regelt, daR etwa 0,01-25 $10^{-5}$ mol Metallionen pro Minute und pro Quadratmeter der zu beschichtenden Oberfläche zugeführt werden.

Gleichzeitig mit der oder den Metallsalzlösung-(en) wird eine Base, vorzugsweise eine wäßrige 0,025-10 mol Alkali- bzw. Ammoniumhydroxydlösung oder auch eine äquivalente Menge gasförmigen Ammoniaks, eingeführt. Als Alkalihydroxide kommen im wesentlichen Natrium- und Kaliumhydroxid in Frage. Die Zuführung der Base wird dabei so gesteuert, daß stets im wesentlichen der pH-Wert gehalten wird, der zu Beginn der Belegung im Bereich von pH 2-6 gewählt worden ist.

Um den pH-Wert konstant zu halten, kann man auch Puffersysteme hinzufügen, z. B. Phosphat-, Acetat-, Citrat- und Glykokollpuffer. Diese können entweder in der Glimmerschuppensuspension vorgelegt oder vorteilhafter mit der Alkali- oder Ammoniumhydroxydlösung zugefügt werden. In vielen Fällen ist aber das Hinzufügen weiterer Fremdionen nicht erwünscht, so daß man vorzieht, den gewünschten pH-Wert durch genaue Eindosierung von Base konstant zu halten.

Die Belegungsdauer kann weitgehend variiert werden. Sie ist im wesentlichen abhängig von der Konzentration der zugeführten Metallsalzlösung, der Oberfläche der zu belegenden Substrate und der Dicke der aufzubringenden Pseudobrookit-Schicht. In der Regel verwendet man Zeiten von etwa 1-24 Stunden.

Das nach dem erfingungsgemäßen Verfahren erhaltene Farbglanzpigment kann nach allen üblichen Methoden aufgearbeitet und aus dem Reaktionsgemisch isoliert werden. Vorteilhaft wird es noch 1/2-4 Stunden unter Rühren in der Suspension etwa 50-100 °C nachgetempert, wodurch die Oxidschicht verfestigt wird. Anschließend wird das Pigment zweckmäßig gewaschen, gegebenenfalls unter vorheriger Einstellung des pH-Wertes auf 5-7. Das Trocknen erfolgt in an sich üblicher Weise bei Temperaturen von etwa 90° bis etwa 150 °C.

Die Gesamtschichtdicke der aufgefällten Schichten bewegt sich, je nach dem gewünschten Farbton vorzugsweise zwischen etwa 30 und etwa 180 nm. Mit zunehmender Schichtdicke geht bekanntlich die Interferenzfarbe kontinuierlich von Blaugrau über Silber, Gold, Orange, Rot, Violett und Blau in Grün über. Anschließend erhält man Interferenzfarben höherer Ordnung. Bevorzugt sind Schichtdicken im Bereich zwischen 30 und 180 nm, da sie Interferenzfarben erster Ordnung entsprechen.

Die erhaltenen Pigmente sind lichtempfindlich und werden deshalb zweckmäßig durch Kalzinieren bei Temperaturen von 700-1100 °C, vorzugsweise 900-1000 °C, in an sich bekannter Weise stabilisiert. Damit werden sie auch beständig gegen Temperatureinflüsse.

Nach dem Trocknen und Glühen, bei dem die Metalloxidschicht weitgehend entwässert ist, erhält man Pigmente, die einen Gehalt an der Pseudobrookit-Schicht von 10 bis 60 %, bezogen auf das Gesamtpigmentgewicht, vorzugsweise von 20 bis 50 %, aufweisen.

In die Pseudobrookit-Schicht können noch Dotierstoffe, insbesondere weitere gefärbte oder farblose Metalloxide, eingebaut werden. Als solche kommen beispielsweise in Frage Verbindungen von Aluminium(III), Silicium(IV), Zinn(IV), Zirkonium(IV), Chrom(III), Bor(III) und Phosphor(V). Diese Dotierstoffe werden gegebenenfalls jeweils in Mengen von 0-2 Gew.-% eingebracht. Insgesamt sollte jedoch eine Menge von 2-5 Gew.-% nicht überschritten werden. Falls Dotierstoffe in die Schicht eingebaut werden sollen, können diese der Glimmersuspension, einer der zugegebenen Salzlösungen oder gegebenenfalls auch der zudosierten Base in Form von wasserlöslichen Salzen beigegeben werden. Die Dotierstoffe sind in der Regel in der Metalloxidschicht homogen verteilt. Es ist aber auch möglich und gegebenenfalls vorteilhaft, eine Anreicherung entweder in Glimmernähe oder an der Oberfläche des Pigments vorzunehmen.

Es ist weiterhin möglich, die Pigmente einer Nachbeschichtung oder Nachbehandlung zu unterziehen, die die Licht-, Wetter-und chemische Stabilität weiter erhöht oder die Handhabung des Pigments, insbesondere die Einarbeitung in unterschiedliche Medien, erleichtert. Als Nachbeschichtung bzw. Nachbehandlung kommen beispielsweise die in den DE-PS 22 15 191, DE-OS 31 51 354, DE-OS 32 35 017 oder DE-OS 33 34 598 beschriebenen Verfahren in Frage. Aufgrund der bereits ohne diese zusätzlichen Maßnahmen sehr guten Eigenschaften der erfindungsgemäßen Pigmente machen diese gegebenenfalls noch aufgebrachten Stoffe nur etwa 0-5, insbesondere 0-3 Gew.-%, des gesamten Pigments aus.

Nach der vorliegenden Erfindung werden besonders vorteilhafte Perlglanzpigmente erhalten, die den bisher bekannten an Farbe und Glanz bei Verwendung in silikatischen Schmelzflüssen bei weitem überlegen sind. So kann ein vorteilhaftes Goldpigment erhalten werden, mit dem nach Einarbeiten in z.B. Glasuren und/oder Emails Artikel erhalten werden, die sich in der Farbe, in Glanz und Schönheit nicht von solchen Artikeln unterscheiden, die mit metallischem Gold eingefärbt worden sind.

Als Fritten werden silikatische Schmelzflüsse literaturbekannter Zusammensetzung und Eigenschaften (z. B. Winnacker-Küchler, Chemische Technologie Bd. 2, Anorg. Technologie II, Carl-Hanse-Verlag, München, 1959) verwendet, vorzugsweise Fritten, deren Schmelzbereich zwischen 500-1100 °C liegt.

Neben den sonst üblichen Anwendungsmöglichkeiten eignen sich die neuen Pigmente insbesondere auch für kosmetische Zwecke, da sie aus physiologisch unbedenklichen Metalloxiden zusammengesetzt sind. Hauptsächlich werden sie jedoch als Pigmente zum Anfärben von z. B. Kunststoffen, kosmetischen Artikeln wie Lippenstiften und Seife, Glas, Keramik, Lacken, Farben sowie Kautschuk und Gummiwaren eingesetzt. Sie eignen sich wegen ihrer Temperaturbeständigkeit auch vor allem für Einbrennlacke, zum Färben von Schmelzflüssen, von Glas und von keramischem Material, das gebrannt wird. In der Regel werden die Pigmente in Mengen bis zu 30 %, vcrzugsweise etwa 0,5-10 %, eingesetzt. In Kunststoffen liegt der Anteil in der Regel relativ niedrig, z. B. bei 0,5-3 %, während in kosmetischen Präparaten z. B. Lippenstiften bis zu 30 %, vorzugsweise 5-25 %, bezogen auf die gesamte Lippenstiftmasse, verwendet werden können.

Die neuen Pigmente zeigen z. B. nach dem Einarbeiten in Kunststoffolien kräftige Farben, die sich auch bei Änderung des Betrachtungswinkels nicht verändern, da diese Farben nicht nur Interferenz-, sondern auch Körperfarben sind. So zeigt z. B. eine Kunststoffolie, die ein goldfarbenes Pigment nach der vorliegenden Erfindung enthält, eine kräftige schöne Goldfarbe, während ein entsprechendes Pigment ohne färbendes Metalloxid in der Aufsicht eine farbschwächere goldgelbe Interferenzfarbe zeigt und in der Durchsicht die hellblaue Komplementärfarbe. Das erfindungsgemäße Pigment weist dagegen auch in der Durchsicht die gelbe Eigenfarbe des Eisen-Titan-Oxids auf.

**Beispiel 1**

Zu einer Suspension von 100 g Kaliglimmer mit einem Teilchendurchmesser von etwa 10-100 $\mu$m in 2000 ml demineralisiertem Wasser werden bei einer Temperatur von 75 °C und einem pH-Wert von 4,0 gleichzeitig 650 ml einer wäßrigen $FeCl_3$-Lösung (152 g $FeCl_3$/l) mit einer Dosiergeschwindigkeit von 1 ml pro Minute und 650 ml einer wäßrigen $TiCl_4$-Lösung (90 g $TiCl_4$/l) mit einer Dosiergeschwindigkeit von 1,0 ml pro Minute zugeben. Nach ca. 1 Stunde werden die Dosiergeschwindigkeiten für beide Lösungen verdoppelt. Während der gesamten Zugabezeit wird der pH-Wert durch gleichzeitiges Zutropfen einer 10 %igen Natronlauge konstant gehalten. Nach 1/2stündigem Nachrühren wird das Pigment abgetrennt, mit Wasser gewaschen, bei 120 °C getrocknet und 30 Minuten bei 900 °C geglüht.

Man erhält ein goldenes, glänzendes Pigment mit hohem Deckvermögen, dessen Farbe und Glanz auch nach Brennen bei Temperaturen zwischen 500° und 1100 °C in Glasuren und Emails erhalten bleibt.

Analytisch findet man ein Molverhältnis Fe : Ti von 2 : 1, und die Röntgenstrukturanalyse ergibt ein Pseudobrookit-Gitter-Typ ($Fe_2 TiO_5$).

**Beispiel 2**

100 g eines nach bekanntem Verfahren mit $SiO_2$ (2 Massen-%) belegten Kaliglimmers mit einem Teilchendurchmesser von 10-100 $\mu$m werden in 2000 ml demineralisiertem Wasser suspendiert. Bei 75 °C und einem pH-Wert von 4,0 werden 1300 ml einer wäßrigen Lösung, die 99 g $FeCl_3$ und 58 g $TiCl_4$ enthält, mit einer Geschwindigkeit von 2 ml pro Minute zudosiert, wobei der pH-Wert durch gleichzeitiges Zutropfen von 10 %iger Natronlauge konstant gehalten wird. Nach 1/2stündigem Nachrühren wird das Pigment abgetrennt, mit Wasser gewaschen, bei 120 °C getrocknet und 30 Minuten bei 900 °C geglüht.

Man erhält ein goldenes, glänzendes Pigment mit hohem Deckvermögen, dessen Farbe und Glanz auch nach Brennen bei Temperaturen zwischen 500° und 1100 °C in Glasuren und Emails erhalten bleibt.

Analytisch findet man ein Molverhältnis Fe : Ti von 2 : 1, und die Röntgenstrukturanalyse ergibt ein Pseudobrookit-Gitter-Typ ($Fe_2 TiO_5$).

**Beispiel 3**

100 g Kaliglimmer werden mit 500 ml einer wäßrigen $FeCl_3$-Lösung (152 g $FeCl_3$/l) und 650 ml einer wäßrigen $TiCl_4$-Lösung (90 g $TiCl_4$/l) analog Beispiel 1 behandelt.

Man erhält ein goldenes, glänzendes Pigment, das in seinem Deckvermögen und seiner thermischen Stabilität dem aus Beispiel 1 kaum nachsteht.

Die Analysen ergeben ein Molverhältnis Fe : Ti von 1,5 : 1 mit einem leicht gestörten Pseudobrookit-Gitter-Typ.

**Beispiel 4**

100 g Kaliglimmer werden mit 1450 ml einer wäßrigen Lösung, die 122 g $FeCl_3$ und 58 g $TiCl_4$ enthält, analog Beispiel 2 behandelt.

Man erhält ein goldenes, glänzendes Pigment, das in seinem Deckvermögen und seiner thermischen Stabilität dem aus Beispiel 1 kaum nachsteht.

Die Analysen ergeben ein Molverhältnis Fe : Ti von 2,5 : 1 mit einem leicht gestörten Pseudobrookit-Gitter-Typ.

**Beispiel 5**

100 g eines nach bekanntem Verfahren mit $Al_2O_3$ (2 Massen-%) belegten Kaliglimmers werden analog Beispiel 2 behandelt.

Man erhält ein goldenes, glänzendes Pigment mit hohem Deckvermögen, dessen Farbe und Glanz auch nach Brennen bei Temperaturen zwischen 500° und 1100 °C in Glasuren und Emails erhalten bleibt.

**Beispiel 6**

100 g eines nach bekannten Verfahren mit $SnO_2$ (2 Massen-%) belegten Kaliglimmer werden analog Beispiel 1 behandelt.

Man erhält ein goldenes, glänzendes Pigment mit hohem Deckvermögen, dessen Farbe und Glanz auch nach Brennen bei Temperaturen zwischen 500° und 1100 °C in Glasuren und Emails erhalten bleibt.

## Beispiel 7

15 g des nach Beispiel 1 hergestellten Farbglanzpigments werden vermischt mit 100 g eines Emailschlickers, der zu 60 Massen-% aus der im Handel erhältlichen Fritte TR 2524 (Fa. Bayer, Leverkusen), 3,5 Massen-% Blauton, 0,2 Massen-% Natriumaluminat, 0,2 Massen-% Kaliumcarbonat und 36,1 Massen-% Wasser besteht. Zu dieser Mischung gibt man 115 ml einer 2 %igen Acrylatlösung (Rohagit SD 15 der Fa. Röhm, Darmstadt) und stellt einen pH-Wert von 10 mittels einer 25%igen Ammoniaklösung ein. Die so erhaltene Mischung wird auf ein bereits grundemailliertes Stahlblech gespritzt, getrocknet und bei 820 ° C 3-4 min gebrannt.

Man erhält ein glänzendes, goldfarbenes Email mit glatter Oberfläche.

## Beispiel 8

2,0 g des nach Beispiel 1 hergestellten Farbglanzpigments werden mit 3,0 g der im Handel erhältlichen Fritte M 204 (Fa. Nitto Shokai) vermischt und mit 8,8 g eines Siebdrucköls angeteigt. Diese Mischung wird im Siebdruckverfahren auf einen vorglasierten Scherben aufgetragen und bei 730 ° C 30 min gebrannt.

Man erhält eine glänzende goldfarbene Glasurschicht mit glatter Oberfläche.

## Patentansprüche

1.  Farbglanzpigmente auf Basis von mit einer Metalloxidschicht überzogenen plättchenförmigen Substraten, insbesondere Glimmer oder mit Metalloxiden überzogener Glimmer, wobei die Metalloxidschicht sowohl Titan als auch Eisen enthält, dadurch gekennzeichnet, daß die Metalloxidschicht im wesentlichen einen Pseudobrookit der Zusammensetzung $(Fe_2O_3)_x$ • $(TiO_2)$, worin x alle Werte zwischen 0,75 und 1,5 annehmen kann, enthält, und wobei in der Metalloxidschicht ggf. noch 0 bis 5 Gew.% Dotierstoffe und auf der Metalloxidschicht ggf. noch 0 bis 5 Gew.% einer Nachbeschichtung enthalten sind.

2.  Verfahren zur Herstellung von Farbglanzpigmenten durch Beschichten von plättchenförmigen Substraten wie Glas, Glimmer oder mit Metalloxiden überzogener Glimmer mit einer Eisen und Titan enthaltenden Metalloxidschicht und anschließendes Glühen, dadurch gekennzeichnet, daß man zu einer wäßrigen Suspension eines plättchenförmigen Substrats bei einer Temperatur zwischen 50 und 100 ° C und einem pH-Wert zwischen 2,0 und 6,0 gleichzeitig eine wäßrige Eisen(III)-salzlösung, eine wäßrige Titan(IV)-salzlösung und eine Base so zuführt, daß stets ein Molverhältnis von Eisen zu Titan wie 1,5-2,5 : 1 eingehalten wird und der pH-Wert während der gesamten Beschichtung nahezu konstant bleibt.

3.  Verfahren nach Anspruch 2, dadurch gekennzeichnet, daß als Titansalz Titantetrachlorid verwendet wird.

4.  Verfahren nach Anspruch 2, dadurch gekennzeichnet, daß als Eisensalz Eisen(III)-chlorid verwendet wird.

5.  Verfahren nach Anspruch 2, dadurch gekennzeichnet, daß als Substrat ein mit $SiO_2$, $SnO_2$, $Al_2O_3$, $TiO_2$ und/oder $Fe_2O_3$ belegter Glimmer verwendet wird.

6.  Verwendung der Farbglanzpigmente nach Anspruch 1-6 in Kunststoffen, Lacken, Kosmetika und silikatischen Schmelzen, wie Emails und Glasuren.

## Claims

1.  Colour lustre pigments based on lamellar substrates coated with a metal oxide layer, in particular mica or mica coated with metal oxides, the metal oxide layer containing both titanium and iron, characterized in that the metal oxide layer contains essentially a pseudobrookite of the composition $(Fe_2O_3)_x$ • $(TiO_2)$ in which x can have any value between 0.75 and 1.5, and may optionally additionally contain 0 to 5% by weight of doping substances and may optionally have been additionally overlaid with 0 to 5% by weight of an after-coating.

2.  Process for the preparation of colour lustre pigments by coating lamellar substrates, such as glass, mica or mica coated with metal oxides, with a metal oxide layer containing iron and titanium and subsequent calcination, characterized in that an agueous iron(III) salt solution, an aqueous titanium(IV) salt solution and a base are added to an aqueous suspension of a lamellar substrate at a temperature between 50 and 100 ° C, a pH between 2.0 and 6.0 and simultaneously, in such a way that a molar ratio of iron to titanium such as 1.5 - 2.5 : 1 is maintained and the pH remains virtually constant during the whole coating operation.

3. Process according to Claim 2, characterized in that titanium tetrachloride is used as the titanium salt.

4. Process according to Claim 2, characterized in that iron(III) chloride is used as the iron salt.

5. Process according to Claim 2, characterized in that a mica coated with $SiO_2$, $SnO_2$, $Al_2O_3$, $TiO_2$ and/or $Fe_2O_3$ is used as the substrate.

6. The use of the colour lustre pigments according to Claim 1-6 [sic] in plastics, paints, cosmetics and silicate melts, such as vitreous enamels and glazes.

**Revendications**

1. Pigments colorés brillants à base de substrats en tablettes revêtus d'une couche d'oxydes métalliques, en particulier à base de mica ou de mica revêtu d'oxydes métalliques, la couche d'oxydes métalliques contenant à la fois du titane et du fer, caractérisés en ce que la couche d'oxydes métalliques contient essentiellement une pseudobrookite de composition $(Fe_2O_3)_x.(TiO_2)$ dans laquelle x peut prendre toutes les valeurs de 0,75 à 1,5, la couche d'oxydes métalliques pouvant éventuellement contenir encore 0 à 5 % en poids d'agents dopants ou porter éventuellement un revêtement complémentaire de 0 à 5 % en poids.

2. Procédé de préparation des pigments colorés brillants par revêtement de substrats en forme de tablettes tels que du verre, du mica ou du mica revêtu d'oxydes métalliques, par une couche d'oxydes métalliques contenant du fer et du titane et calcination subséquente, caractérisé en ce que, à une suspension aqueuse d'un substrat sous forme de tablettes, à une température de 50 à 100°C et un pH de 2,0 à 6,0, on ajoute simultanément une solution aqueuse de sel de fer-III, une solution aqueuse de sel de titane-IV et une base en maintenant en permanence un rapport molaire fer/titane de 1,5 à 2,5:1 et en maintenant le pH pratiquement constant pendant tout le revêtement.

3. Procédé selon la revendication 2, caractérisé en ce que le sel de titane utilisé est le tétrachlorure de titane.

4. Procédé selon la revendication 2, caractérisé en ce que le sel de fer utilisé est le chlorure de fer-III.

5. Procédé selon la revendication 2, caractérisé en ce que le substrat utilisé est un mica revêtu de $SiO_2$, de $SnO_2$, d'$Al_2O_3$, de $TiO_2$ et/ou $Fe_2O_3$.

6. Utilisation des pigments colorés brillants selon les revendications 1 à 6 dans des résines synthétiques, des vernis, des produits cosmétiques et des masses de fusion silicatées telles que des émaux et des glaçures.